# EUROPEAN PATENT SPECIFICATION

(11) **EP 1 308 155 B1**
(45) Date of publication and mention of the grant of the patent: **21.06.2006**
(21) Application number: 02022911.8
(22) Date of filing: 10.10.2002
(51) Int. Cl.: A61K 9/08, A61K 33/26, A61K 47/36, A61K 47/48, A61P 7/06

(54) **Injectable solution containing a colloid of iron and shark cartilage-derived chondroitin sulfate**
Injektionslösung enthaltend ein Kolloid aus Eisen und Haiknorpel-Chondroitinsulfat
Solution injectable contenant un colloide de fer et chondroitine sulfate de cartilage de requin

(30) Priority: 12.10.2001 JP 2001314977
(43) Date of publication of application: 07.05.2003
(73) Proprietor: NIPRO CORPORATION, Kita-ku, Osaka 531-8510 (JP)
(72) Inventor: Nishida, Seiji, Kita-ku, Osaka 531-8510 (JP); Katayama, Naohisa, Kita-ku, Osaka 531-8510 (JP); Katsumata, Takashi, Kita-ku, Osaka 531-8510 (JP); Sato, Makoto, Kita-ku, Osaka 531-8510 (JP)
(74) Representative: Albrecht, Thomas

(56) References cited:
- WO-A-93/09766
- FR-A- 2 454 447
- FR-A- 2 804 022
- SIPOS, P. ET AL.: "Rod-like iron(III) oxyhydroxide particles in iron(III)-polysaccharide solutions" JOURNAL OF INORGANIC BIOCHEMISTRY, vol. 58, no. 2, 1995, pages 129-138, XP002234021
- KATO, T. ET AL.: "Chondroitin sulfate iron colloid as an MR contrast agent for the hepatic reticuloendothelial system" JOURNAL OF COMPUTER ASSISTED TOMOGRAPHY, vol. 17, no. 4, 1993, pages 603-608, XP009006954
- ABDATA PHARMA-DATEN-SERVICE: "Pharmazeutische Stoffliste, 13. Auflage" 2002 , WERBE- UND VERTRIEBSGESELLSCHAFT DEUTSCHER APOTHEKER M.B.H , ESCHBORN/TAUNUS XP002234022 See C-Ch (4/2002), page 453, ABDATA-Nr.: 61907
- BARONE, D. ET AL.: "Ferric chondroitin 6-sulfate (Condrofer(R)): A new potent antianaemic agent with a favourable pharmacokinetic profile" DRUGS EXPTL. CLIN. RES., SUPPL. 1, vol. XIV, 1988, pages 1-14, XP009007325
- RONCA, G. ET AL.: "Metabolic fate of partially depolymerized shark chondroitin sulfate in man" INT. J. CLIN. PHARM. RES., vol. XIII (Suppl.), 1993, pages 27-34, XP009006912
- CHEMICAL ABSTRACTS, vol. 108, no. 10, 7 March 1988 (1988-03-07) Columbus, Ohio, US; abstract no. 81913, RODRIGUEZ, D. ET AL.: "Preparation of chondroitin sulfate from shark cartilage" XP002150348 & REV. CUBANA FARM., vol. 21, no. 2, 1987, pages 133-140, Havana, CUBA
- CHEMICAL ABSTRACTS, vol. 116, no. 11, 16 March 1992 (1992-03-16) Columbus, Ohio, US; abstract no. 104510, "Manufacture of low-molecular weight chondroitin sulfuric acid with chondroitinase" XP002150347 & JP 03 247296 A (RESEARCH DEVELOPMENT CORP. OF JAPAN) 5 November 1991 (1991-11-05)

## Description

### Technical Field of the Invention

The present invention relates to an iron preparation capable of preventing and treating the symptoms of iron-deficiency anemia in humans and mammals. More specifically, the present invention relates to an injectable solution with excellent safety and pharmaceutical stability for the supply of chondroitin sulfate iron colloid.

### Background of the Invention

Iron is one of essential metallic nutrients for humans and mammals. If a deficiency of iron is caused by an insufficient uptake of iron by oral administration, bleeding, or the like, the supply of iron becomes absolutely indispensable. When iron is parenterally supplied, there is a problem in terms of toxicity because an ionic iron compound binds to transferrin and also binds to plasma protein, causing shock or the like. Thus, there is a need to devise the supplying of iron in a colloidal form with less side effects. For an iron ion to be parenterally supplied to humans, ferric chloride is used in general. In a solution, such ferric chloride exists as a ferric hydroxide colloid particle. Such a colloid particle includes oxy chloride (FeOCl) in addition to ferric oxide (Fe₂O₃) and water, and oxy chloride dissociates to FeO⁺ and Cl⁻. As a result, the colloid particle becomes a hydrophobic colloid which is positively charged and has a tendency to aggregate. If the pH value thereof rises to about 3 or more, it will precipitate out of solution as a result of the aggregation ("Colloid Chemistry", written by B. Jirgensons et al., and translated under the editorship of Fumikazu Tamamushi, Baifukan, Tokyo, 1967, Japan).

Heretofore, an iron hydroxide colloid solution in which dextran is used as a protective colloid has been used in the United States, while iron-poly(sorbitol gluconic acid) complex salt has been used in Europe (Goodman and Gilman: The Pharmacological Basis of Therapeutics, MacMillan, New York 1980, pp1325-1326). In Japan, on the other hand, an iron colloid solution, in which chondroitin sulfate having a high iron utilization ratio and less side effects is used as a protective colloid, has been used. For example, in Japan, chondroitin sulfate iron colloid is commercially available as an intravenous injection preparation for iron deficiency anemia, Blutal (trade name, Dainippon Pharmaceutical Co., Ltd. Japan). In addition, a preparation containing chondroitin sulfate iron colloid as a supplement of essential trace elements of total parenteral nutrition is also commercially available as Elemenmic injection, Elemate injection (trade names, Ajinomoto Pharma, Co., Ltd. Japan), Mineralin injection, Parmirin injection (tradenames, Nippon Pharmaceutical Co., Ltd. Japan/Takeda Chemical Industries, Ltd. Japan), Elemeal injection (trade name, Sawai Pharmaceutical, Co., Ltd. Japan), and Volvix injection (trade name, Fujiyakuhin Co., Ltd. Japan/Yakult Honsha Co., Ltd. Japan).

Each of these preparations containing chondroitin sulfate iron colloid, commercially available in Japan, is prepared using a bovine sodium chondroitin sulfate as a protective colloid. In 1996, it was announced in Britain that there was a relationship between the onset in a patient of Variant Creutzfeldt-Jakob disease (vCJD) and bovine spongiform encephalopathy (BSE). An abnormal prion protein regarded as a cause of BSE is heat stable, so that a high-temperature, high-pressure treatment and an alkali treatment will not perfectly deactivate the prion protein.

In consideration of the outbreak trend of bovine BSE in Europe, for any drug or the like manufactured using a raw material derived from cows or the like, there is a need that manufacturers and so on take measures for ensuring quality and safety. Bovine chondroitin sulfate has been isolated and purified from bovine tracheae. However, there is a demand to use a safer and more secure chondroitin sulfate.

### Summary of the Invention

The present invention provides an injectable solution which comprises a shark-derived chondroitin sulfate iron colloid not containing any BSE causative agent, which is safe and excellent in pharmaceutical stability, and in addition is stable when it is mixed in infusions such as hyperalimentation preparations or the like.

After intensively studying various ways of obtaining an injectable solution with excellent pharmaceutical stability and stability when mixed in infusions such as hyperalimentation preparations or the like, the inventors have found that a shark-derived chondroitin sulfate iron colloid solution, in which shark-derived sodium chondroitin sulfate is provided as a protective colloid, has an excellent stability after the application of heat. By devoting themselves to research depending on these findings, the present invention has finally been completed.

That is, the present invention relates to:
(1) an injectable solution which comprises a shark-derived chondroitin sulfate iron colloid,
(2) an injectable solution as described in item (1), wherein the shark-derived chondroitin sulfate iron colloid is prepared from a shark-derived chondroitin sulfate,
(3) an injectable solution as described in item (2), wherein the shark-derived chondroitin sulfate is an alkali metal salt of shark-derived chondroitin sulfate,
(4) an injectable solution as described in item (2), wherein the shark-derived chondroitin sulfate is a shark-derived sodium chondroitin sulfate,
(5) an injectable solution as described in item (4), wherein the shark-derived sodium chondroitin sulfate has an average molecular weight of from about 10,000 to about 25,000, a limiting viscosity of from about 0.27 to about 0.65 dL/g, and a sulfur content of from about 6.4 to about 7.0 W/W %,
(6) an injectable solution as described in item (5), wherein the weight ratio of iron : sodium chondroitin sulfate is 1:7 or less.
(7) an injectable solution as described in item (1), wherein the shark-derived chondroitin sulfate iron colloid is produced by adding an aqueous ferric salt solution and an aqueous alkali metal hydroxide solution to an aqueous shark-derived chondroitin sulfate solution and maintaining the pH value of the resulting mixture in the range of from about 5.5 to about 7.5,
(8) a method for manufacturing an injectable solution, which comprises adding an aqueous ferric salt solution and an aqueous alkali metal hydroxide salt solution to an aqueous shark-derived chondroitin sulfate solution and maintaining the pH value of the resulting mixture in the range of from about 5.5 to about 7.5, and
(9) a method for manufacturing an injectable solution, which comprises adding an aqueous ferric salt solution and an aqueous sodium hydroxide solution to a shark-derived sodium chondroitin sulfate solution and maintaining the pH value of the resulting mixture in the range of from about 5.5 to about 7.5.

### Detailed Description of the Invention

An injectable solution of the present invention, which includes a shark-derived chondroitin sulfate iron colloid, can be manufactured by adding an aqueous ferric salt solution and an aqueous alkali metal hydroxide solution to an aqueous shark-derived chondroitin sulfate solution so that the resulting mixture has a pH value adjusted to any pH value within the range of from about 5.5 to about 7.5.

The shark-derived chondroitin sulfate is, for example, an alkali metal salt such as a sodium salt or potassium salt of shark-derived chondroitin sulfate, and preferably, shark-derived sodium chondroitin sulfate. The shark-derived sodium chondroitin sulfate may be, for example, one which is derived from shark cartilage and has an average molecular weight of from about 10,000 to about 25,000,a limiting viscosity of from about 0.27 to about 0.65 dL/g (based on viscosity measurement by capillary tube viscometer), and a sulfur content of from about 6.4 to about 7.0 W/W %. Preferably, the shark-derived sodium chondroitin sulfate is one in which a composition ratio of chondroitin-4-sulfate (chondroitin sulfate A): chondroitin-6-sulfate (chondroitin sulfate C) is about 1 : 3.

The chondroitin sulfate is a linear polymeric polysaccharide having a repetitive structure with disaccharide units of [→ 4-glucuronic acid β1 → 3N-acetyl-D-galactosamine β1 →] and is a poly anion having a high negative charge in which the isomers present depend on the number of sulfate groups bound to such disaccharide units and the binding positions thereof. Table 1 shows a comparison of the isomer-composition ratio of sodium chondroitin sulfate (an average molecular weight of 20,000 to 25,000) derived from each of shark cartilages and from bovine tracheae.

**Table 1**

| Position of sulfate group sulfate group | Isomer composition ratio | |
|---|---|---|
| | Shark-cartilage-derived Chs Molecular weight 20,000-25,000 | Bovine-trachea-derived Chs Molecular weight 20,000-25,000 |
| ΔDi-0S | 4.4% | 5.1% |
| ΔDi-4S | 21.0% | 47.5% |
| ΔDi-6S | 60.4% | 43.0% |
| ΔDi-diS_{D} | 12.1% | 1.1% |
| ΔDi-diS_{E} | 2.0% | 0.7% |

| | | |
|---|---|---|
| (Note) Chs: sodium chondroitin sulfate; ΔDi-0S: a sulfate group is not bonded; ΔDi-4S: a sulfate group is bonded to C-4 position of N-acetyl-D-galactosamine; ΔDi-6S: a sulfate group is bonded to C-6 position of N-acetyl-D-Galactosamine; ΔDi-diS_{D} : a sulfate group is bonded to C-6 position of N-acetyl-D-galactosamine, and a sufate group is bonded to C-2 position of D-glucuronic acid; ΔDi-diS_{E} : Sulfate groups are bonded to C-4 and C-6 positions of N-acetyl-D-galactosamine. | | |

The ferric salt of the ferric slat solution is a compound containing ferric iron which can be used in the body, for example, ferric chloride hexahydrate (FeCl₃·6H₂O), ferric citrate (FeC₆H₅O₇), iron oxyhydroxide (FeO(OH)), iron nitrate enneahydrate (Fe(NO₃)₃·9H₂O), iron oxide (Fe₂O₃), iron sulfate (Fe₂(SO₄)₃·nH₂O), iron phosphate (FePO₄·nH₂O), or the like. Ferric salt changes to ferric hydroxide in an aqueous solution, and the shark-derived chondroitin sulfate is used as a protective colloid of the hydrophobic colloid solution thereof. Among others, ferric chloride such as ferric chloride hexahydrate (FeCl₃·6H₂O) or the like is preferable. The weight ratio of iron: shark-derived sodium chondroitin sulfate is 1 : 7 or less.

The alkali metal hydroxide is, for example, sodium hydroxide or potassium hydroxide, preferably sodium hydroxide.

In the manufacturing method of the present invention, an appropriate amount of the aqueous solution of ferric salt (e.g., ferric chloride hexahydrate) in a water for injection and an appropriate amount of the aqueous solution of alkali metal hydroxide (e.g., sodium hydroxide) are added with stirring to an aqueous solution of shark-derived chondroitin sulfate (e.g., sodium chondroitin sulfate) in a water for injection corresponding to the above weight ratio to iron. It is preferable to adjust the pH value of the reaction mixture to any constant pH value within the range of from about 5.5 to about 7.5.

The concentration of ferric salt (e.g., ferric chloride hexahydrate) in the water for injection is generally in the range of from about 3 to about 62 W/V %, preferably about 13 to about 32 W/V %.

The concentration of alkali metal hydroxide (e.g., sodium hydroxide) in the aqueous solution to be added is generally in the range of from about 1 to about 28 W/V %, preferably from about 2 to about 7 W/V %.

The concentration of chondroitin sulfate in the aqueous chondroitin sulfate solution is generally in the range of from about 3 to about 30 W/V %, preferably about 4 to about 20 W/V %.

The reaction mixture containing the ferric salt, the alkali metal hydroxide and the chondroitin sulfate is stirred sufficiently to maintain the pH value of the mixture at a predetermined value.

The reaction time may be appropriately selected by a person skilled in the art. In general, however it is about 1 hour to about 6 hours. The reaction temperature may be appropriately selected by a person skilled in the art. Preferably it is about 5°C to about 25°C.

The thus-obtained solution containing a shark-derived chondroitin sulfate iron colloid may be used as an injection after sterilization, if required. In addition, containers can each be filled with a small limited amount of the solution (e.g., 1, 2, or 4 ml each), and then sealed and subjected to sterilization (e.g., high-pressure steam sterilization). For the injectable solution of the present invention, it is preferable that the pH value is in the range of from about 5.0 to about 7.5.

For the container for the injectable solution of the present invention, for example, a glass container (such as an ampule), and a container made of a plastic material such as polypropylene, including a pre-filled syringe type, can be used.

The injectable solution of the present invention can be administered to humans or mammals safely, while scarcely causing any side effects, in accordance with per se known methods. The amount of iron contained in the injectable solution of the present invention to be administered is in the range of from about 0.9 to about 720 µmol, preferably from about 9 to about 720 µmol, in about 2 to about 20 ml of the aqueous solution. The injectable solution of the present invention may optionally include an additional element such as copper, zinc, manganese, selenium, iodine, and chromium. In this case, the injectable solution of the present invention to be administered preferably includes, as a daily amount per an adult person, from about 0.9 to about 55 µmol of copper, from about 3.85 to about 210 µmol of zinc, from 0 to about 51 µmol of manganese, from about 0.025 to about 5.0 µmol of selenium, and from 0 to about 11 µmol of iodine and, more preferably, from about 9.1 to about 27.3 µmol of copper, from about 38.5 to about 61.5 µmol of zinc, from 0 to about 14.5 µmol of manganese, from about 0.25 to about 2. 5 µmol of selenium, and from about 0.6 to about 1.1 µmol of iodine.

### Examples

Hereinafter, the present invention will be described more specifically with reference to examples. In the specification, W/V % means weight/volume %, and W/W % means weight/weight %.

### Example 1

An aqueous ferric chloride solution (20.8 W/V %) and an aqueous sodium hydroxide solution (4.3 W/V %) were fed into separate solutions (6.4 W/V %) of shark-cartilage-derived sodium chondroitin sulfate at weight ratios of iron: sodium chondroitin sulfate of 1:5, 1:10 and 1:12 with stirring at 5 to 25°C for about 60 minutes, while keeping the pH value at about 6.5. Consequently, the resulting chondroitin sulfate iron colloid solutions were diluted with purified water to obtain the object chondroitin sulfate iron colloid solutions with 4 mg/ml of iron concentration.

Each of the thus-obtained solutions was filled into 2-ml glass ampules, followed by melt sealing. Subsequently, ampules of each of the solution were subjected to a high-pressure steam sterilization under the conditions of 105°C for 20 min., 110°C for 20 min., 115°C for 20 min., and 121°C for 20 min., respectively, to obtain samples. This procedure was repeated 5 times.

For each of the samples, the property thereof was observed, followed by the conducting of a filtration test on 10 ml of the sample using a membrane filter (0.2 µm in pore size). The obtained results are shown in Table 2.

Two kinds of the shark-cartilage-derived sodium chondroitin sulfate (Chs), one with a molecular weight of about 10,000 and the other with a molecular weight of 20,000 to 25,000, were examined.

**Table 2**

| Molecular weight of Chs | (Fe:Chs) Weight ratio | Evaluation item | Heat conditions | | | |
|---|---|---|---|---|---|---|
| | | | 105°C 20 minutes | 110°C 20 minutes | 115°C 20 minutes | 121°C 20 minutes |
| 20,000-25,000 | 1:5 | Property | C | C | C | C |
| | | Filtration test | X | O | O | O |
| | 1:10 | Property | A | A | A | A |
| | | Filtration test | O | O | O | O |
| | 1:12 | Property | A | A | A | A |
| | | Filtration test | O | O | O | O |
| 10,000 | 1:5 | Property | B | B | B | B |
| | | Filtration test | O | O | O | O |
| | 1:10 | Property | A | A | A | A |
| | | Filtration test | O | O | O | O |
| | 1:12 | Property | A | A | A | A |
| | | Filtration test | O | O | O | O |

| | | | | | | |
|---|---|---|---|---|---|---|
| (Note) Chs: sodium chondroitin sulfate Contents of evaluation Property: A: Clear dark reddish brown B: Slightly clouded dark reddish brown C: Clearly clouded dark reddish brown Filtration test: O: Passing through the filter X: Not passing through the filter | | | | | | |

As shown in Table 2, it was confirmed that chondroitin sulfate iron colloid solutions prepared with the respective weight ratio of iron : chondroitin sodium sulfate of 1 : 10 and 1 : 12 were stable under the respective heat conditions without causing any precipitates of insoluble contaminants or the like in the property and filtration tests.

### Example 2

An aqueous ferric chloride solution (20.8 W/V %) and an aqueous sodium hydroxide solution (4.3W/V%) were fed into separate solutions of shark-cartilage-derived sodium chondroitin sulfate* at weight ratios of iron: sodium chondroitin sulfate of 1:7, 1:9, 1:11, 1:13 and 1:20 with stirring at 5 to 25°C for about 60 minutes, while keeping the pH value at about 6.5. The resulting chondroitin sulfate iron colloid solutions were diluted with purified water to obtain the object chondroitin sulfate iron colloid solutions with 4 mg/ml of iron concentration.

***Concentration of sodium chondroitin sulfate**

| Iron : sodium chondroitin sulfate | Concentration W/V % of sodium chondroitin sulfate |
|---|---|
| 1:7, 1:9 | 6.4 |
| 1:11 | 7.8 |
| 1:13 | 9.1 |
| 1:20 | 13.7 |

Each of the thus-obtained solutions was filled into 2-ml glass ampules, followed by melt sealing. Subsequently, ampules of each of the solution were subjected to a high-pressure steam sterilization under the conditions of 110°C for 20 min. and 121°C for 20 min., respectively, to obtain samples.

For each of the samples, the property thereof was observed and a filtration test was conducted according to the same conditions as in Example 1. The obtained results are shown in Table 3.

The shark-cartilage-derived sodium chondroitin sulfate (Chs) examined had a molecular weight of from about 10,000.

As shown in Table 3, it was confirmed that chondroitin sulfate iron colloid solutions prepared with respective weight ratios of iron : sodium chondroitin sulfate of 1 : 7 to 20 under the respective heat conditions were stable in the property and filtration tests.

**Table 3**

| Molecular weight of Chs | (Fe:Chs) Weight ratio | Evaluation Evaluation item | Heat condition | |
|---|---|---|---|---|
| | | | 110°C 20 minutes | 121°C 20 minutes |
| 10,000 | 1:7 | Property | A | A |
| | | Filtration test | O | O |
| | 1:9 | Property | A | A |
| | | Filtration test | O | O |
| | 1:11 | Property | A | A |
| | | Filtration test | O | O |
| | 1:13 | Property | A | A |
| | | Filtration test | O | O |
| | 1:20 | Property | A | A |
| | | Filtration test | O | O |

| | | | | |
|---|---|---|---|---|
| (Note) Chs: sodium chondroitin sulfate | | | | |

### Example 3

An aqueous ferric chloride solution (20.8 W/V%) and an aqueous sodium hydroxide solution (4.3 W/V %) were fed into separate solutions (6.4 W/V %) of shark-cartilage-derived sodium chondroitin sulfate* at weight ratios of iron: sodium chondroitin sulfate of 1:5, 1:7, 1:9, 1:11, 1:13 and 1:20 with stirring at 5 to 25°C for about 60 minutes, while keeping the pH value at about 6.5. The resulting chondroitin sulfate iron colloid solutions were diluted with purified water to obtain the object chondroitin sulfate iron colloid solutions with 4 mg/ml of iron concentration.

***Concentration of sodium chondroitin sulfate**

| Iron : sodium chondroitin sulfate | Concentration W/V % of sodium chondroitin sulfate |
|---|---|
| 1:5, 1:7, 1:9 | 6.4 |
| 1:11 | 7.8 |
| 1:13 | 9.1 |
| 1:20 | 13.7 |

Two ml each of an aqueous solution of shark-cartilage-derived chondroitin sulfate iron colloid were filled into a glass ampule (2 ml), followed by melt sealing.

Sealed ampules were subjected to a high-pressure steam sterilization under the conditions of 110°C for 20 min.

The stability test of samples in these ampules were conducted at 70°C. The obtained results are shown in Table 4.

As shown in Table 4, it was confirmed that chondroitin sulfate iron colloid solutions prepared with the respective weight ratio of iron: sodium chondroitin sulfate of 1 : 5 to 20 were stable in the tests of the insoluble contaminant and the iron content as a percentage of the initial content.

**Table 4**

| Evaluation items | (Fe:Chs) Weight ratio | Term of storage | | | |
|---|---|---|---|---|---|
| | | Initial | After 10 days | After 20 days | After 31 days |
| Insoluble cotaminant | 1:5 | none | none | none | none |
| | 1:7 | none | none | none | none |
| | 1:9 | none | none | none | none |
| | 1:11 | none | none | none | none |
| | 1:13 | none | none | none | none |
| | 1:20 | none | none | none | none |
| Iron content (% per the initial content) | 1:5 | 100 | 98.0 (97.5-98.5) | 99.6 (99.0-100.4) | 99.4 (99.1-99.6) |
| | 1:7 | 100 | 101.5 (100.6-102.9) | 100.1 (99.0-101.2) | 100.4 (99.9-101.0) |
| | 1:9 | 100 | 101.6 (100.8-102.1) | 100.3 (99.7-100.6) | 99.9 (99.3-100.4) |
| | 1:11 | 100 | 103.0 (103.0-103.1) | 101.1 (100.8-101.4) | 101.7 (100.8-102.3) |
| | 1:13 | 100 | 101.0 (100.7-101.3) | 99.8 (99.4-100.2) | 99.0 (98.8-99.2) |
| | 1:20 | 100 | 100.9 (100.1-101.5) | 99.9 (99.1-101.3) | 100.6 (99.4-101.8) |

| | | | | | |
|---|---|---|---|---|---|
| (Note) Iron content shows the mean(n=3) and minimum-maximum values as parenthesis. | | | | | |

It was confirmed from the test results in Examples 1 to 3 that chondroitin sulfate iron colloid solutions prepared with a respective weight ratio of iron : sodium chondroitin sulfate of 1 : 7 or less were stable.

### Example 4

A shark-cartilage-derived chondroitin sodium sulfate iron colloid solution in which the weight of sodium chondroitin sulfate was 12 times higher than that of iron (Fe), prepared in Example 1 was filled in a 2-ml glass ampule, followed by melt sealing. Subsequently, the glass ampule was subjected to a high-pressure steam sterilization under the conditions of 110°C for 20 minutes to obtain a sample. Then, 1 ml of the solution was mixed in a commercially available infusion described below, followed by conducting an incompatibility test.

Two kinds of the shark-cartilage-derived sodium chondroitin sulfate (Chs), one with a molecular weight of about 10,000 and the other with a molecular weight of from 20,000 to 25,000, were investigated. That is, the observation was performed with respect to the pH value, and the insoluble contaminants before mixing, just after mixing and 24 hours after mixing. The results are shown in Table 5. The commercially available infusion used was "AMINOTRIPA No.2" (trade name, Otsuka Pharmaceutical Co., Ltd. Japan) and "PNTWIN-3" (trade name, Ajinomoto Pharma Co., Ltd. Japan).

**Table 5**

| Infusion | Molecular weight of Chs | Test item | Before mixing | Just after mixing | 24 hours after mixing |
|---|---|---|---|---|---|
| AMINOTRIPA No.2 | 20,000-25,000 | Property | Clear and colorless | Clear yellowish brown | Clear yellowish brown |
| | | pH value | 5.55 | 5.54 | 5.50 |
| | | Insoluble contaminant | None | None | None |
| | 10,000 | Property | Clear and colorless | Clear yellowish brown | Clear yellowish brown |
| | | pH value | 5.53 | 5.55 | 5.50 |
| | | Insoluble contaminant | None | None | None |
| PNTWIN-3 | 20,000-25,000 | Property | Clear and colorless | Clear yellowish brown | Clear yellowish brown |
| | | pH value | 5.16 | 5.16 | 5.13 |
| | | Insoluble contaminant | None | None | None |
| | 10,000 | Property | Clear and colorless | Clear yellowish brown | Clear yellowish brown |
| | | pH value | 5.16 | 5.16 | 5.14 |
| | | Insoluble contaminant | None | None | None |

As shown in Table 5, each of the injectable solutions (Example 4) of the present invention did not show any change in its properties up to 24 hours after mixing in the commercially available infusion, and precipitates of insoluble contaminants or the like were not observed.

### Effects of the Invention

The injectable solution of the present invention can be safely applied in clinical use without worry of BSE infection. It is pharmaceutically stable and is stable in an infusion such as an intravenous hyperalimentation preparation and the like.

## Claims

1. An injectable solution which comprises a shark-derived chondroitin sulfate iron colloid and an additional element selected from the group consisting of copper, zinc, manganese, selenium, iodine and chromium.

2. An injectable solution as claimed in claim 1, which includes, as a daily amount per an adult person, from about 0.9 to about 55 µmol of copper, from about 3.85 to about 210 µmol of zinc, from 0 to about 51 µmol of manganese, from about 0.025 to about 5.0 µmol of selenium and from about 0 to about 11 µmol of iodine.

3. An injectable solution as claimed in claim 1, wherein the shark-derived chondroitin sulfate iron colloid is prepared from a shark-derived chondroitin sulfate.

4. An injectable solution as claimed in claim 3, wherein the shark-derived chondroitin sulfate is an alkali metal salt of shark-derived chondroitin sulfate.

5. An injectable solution as claimed in claim 3, wherein the shark-derived chondroitin sulfate is a shark-derived sodium chondroitin sulfate.

6. An injectable solution as claimed in claim 5, wherein the shark-derived sodium chondroitin sulfate has an average molecular weight of from about 10,000 to about 25,000, a limiting viscosity of from about 0.27 to about 0.65 dL/g, and a sulfur content of from about 6.4 to about 7.0 W/W %.

7. An injectable solution as claimed in claim 6, wherein the weight ratio of iron : sodium chondroitin sulfate is 1:7 or less.

8. An injectable solution as claimed in claim 1, wherein the shark-derived chondroitin sulfate iron colloid is produced by adding an aqueous ferric salt solution and an aqueous alkali metal hydroxide solution to an aqueous shark-derived chondroitin sulfate iron solution and maintaining the pH value of the resulting mixture in the range of from about 5.5 to about 7.5.

## Patentansprüche

1. Injizierbare Lösung, umfassend ein Chondroitinsulfateisenkolloid von Haien und ein zusätzliches Element, ausgewählt aus der Gruppe, bestehend aus Kupfer, Zink, Mangan, Selen, Jod und Chrom.

2. Injizierbare Lösung nach Anspruch 1, welche als tägliche Menge pro erwachsener Person enthält: etwa 0,9 bis etwa 55 µmol Kupfer, etwa 3,85 bis etwa 210 µmol Zink, 0 bis etwa 51 µmol Mangan, etwa 0,025 bis etwa 5,0 µmol Selen und etwa 0 bis etwa 11 µmol Jod.

3. Injizierbare Lösung nach Anspruch 1, wobei das Chondroitinsulfateisenkolloid von Haien hergestellt ist aus Chondroitinsulfat von Haien.

4. Injizierbare Lösung nach Anspruch 3, wobei das Chondroitinsulfat von Haien ein Alkalimetallsalz von Chondroitinsulfat von Haien ist.

5. Injizierbare Lösung nach Anspruch 3, wobei das Chondroitinsulfat von Haien ein Natriumchondroitinsulfat von Haien ist.

6. Injizierbare Lösung nach Anspruch 5, wobei das Natriumchondroitinsulfat von Haien ein mittleres Molekulargewicht von etwa 10.000 bis etwa 25.000, eine Grenzviskosität von etwa 0,27 bis etwa 0,65 dL/g und einen Schwefelgehalt von etwa 6,4 bis etwa 7,0 Gew.-% besitzt.

7. Injizierbare Lösung nach Anspruch 6, wobei das Gewichtsverhältnis von Eisen : Natriumchondroitinsulfat 1 : 7 oder weniger beträgt.

8. Injizierbare Lösung nach Anspruch 1, wobei das Chondroitinsulfateisenkolloid von Haien hergestellt worden ist durch Zugeben einer wässrigen Eisen(III)-Salzlösung und einer wässrigen Alkalimetallhydroxidlösung zu einer wässrigen Lösung von Chondroitinsulfateisen von Haien und Einstellen des pH-Werts des resultierenden Gemischs auf einen Wert im Bereich von etwa 5,5 bis etwa 7,5.

## Revendications

1. Solution injectable qui comprend un colloïde de fer et chondroïtine sulfate dérivé de cartilage de requin et un élément supplémentaire choisi dans le groupe comprenant le cuivre, le zinc, le manganèse, le sélénium, l'iode et le chrome.

2. Solution injectable selon la revendication 1, qui comprend en quantité quotidienne pour une personne adulte, environ 0,9 à environ 55 µmoles de cuivre, environ 3,85 à environ 210 µmoles de zinc, 0 à environ 51 µmoles de manganèse, environ 0,025 à environ 5,0 µmoles de sélénium et environ 0 à environ 11 µmoles d'iode.

3. Solution injectable selon la revendication 1, dans laquelle le colloïde de fer et chondroïtine sulfate dérivé de cartilage de requin est préparé à partir d'un chondroïtine sulfate dérivé de cartilage de requin.

4. Solution injectable selon la revendication 3, dans laquelle le chondroïtine sulfate dérivé de cartilage de requin est un sel de métal alcalin de chondroïtine sulfate dérivé de cartilage de requin.

5. Solution injectable selon la revendication 3, dans laquelle le chondroïtine sulfate dérivé de cartilage de requin est un chondroïtine sulfate sodique dérivé de cartilage de requin.

6. Solution injectable selon la revendication 5, dans laquelle le chondroïtine sulfate sodique dérivé de cartilage de requin présente un poids moléculaire moyen d'environ 10 000 à environ 25 000, un indice limite de viscosité d'environ 0,27 à environ 0,65 dl/g et une teneur en soufre d'environ 6,4 à environ 7,0 % en poids.

7. Solution injectable selon la revendication 6, dans laquelle le rapport en poids fer : chondroïtine sulfate sodique est de 1:7 ou inférieur.

8. Solution injectable selon la revendication 1, dans laquelle le colloïde de fer et chondroïtine sulfate dérivé de cartilage de requin est produit par addition d'une solution aqueuse de sel ferrique et d'une solution aqueuse d'hydroxyde de métal alcalin, à une solution aqueuse de fer et chondroïtine sulfate dérivé de cartilage de requin et en conservant le pH du mélange obtenu dans une plage d'environ 5,5 à environ 7,5.
